# EUROPÄISCHE PATENTSCHRIFT

(11) **EP 0 205 997 B1**
(45) Veröffentlichungstag und Bekanntmachung des Hinweises auf die Patenterteilung: **03.03.1993**
(21) Anmeldenummer: 86107421.9
(22) Anmeldetag: 02.06.1986
(51) Int. Cl.: A61L 27/00

(54) **Implantat**
Implant
Implant

(30) Priorität: 18.06.1985 DE 3521684
(43) Veröffentlichungstag der Anmeldung: 30.12.1986
(73) Patentinhaber: Anawa München Aktiengesellschaft Biologische Laboratorien, D-82152 Planegg (DE)
(72) Erfinder: Müller-Lierheim, Wolfgang G.K., Dr. rer. nat., D-8032 Gräfelfing (DE)
(74) Vertreter: Nöth, Heinz, Dipl.-Phys.

(56) Entgegenhaltungen:
- EP-A- 0 049 341
- EP-A- 0 146 329
- FR-A- 2 554 349

## Beschreibung

Die Erfindung betrifft ein Implantat mit einem Grundkörper, der einen Oberflächenüberzug aus einem bioaktiven Werkstoff aufweist.

Biokompatibilität und mechanische Festigkeit sind wesentliche Voraussetzungen für einen Implantatwerkstoff. Es sind verschiedene Implantatwerkstoffe bekannt, die in dem sie umgebenden Gewebe unterschiedliche Reaktionen auslösen. So zeigen beispielsweise Metalle, wie Kupfer, Kobalt, Nickel oder Vanadium toxische Reaktionen. Kompatible Implantatwerkstoffe auf Co- oder PMMA-Basis zeigen bindegewebige Abkapselung und Distanzosteogenese. Vitale Implantatwerkstoffe, z. B. Ti-Legierungen, Al₂O₃-Keramik zeigen zwar keine Reaktion, jedoch ergibt sich eine durch die Biomechanik bedingte Abscheidung. Von bioaktiven Werkstoffen erwartet man, daß sie im Körper eine positive Reaktion hervorrufen. Zum einen sollen sie das Knochenwachstum fördern oder beschleunigen, zum anderen soll ein echter Verbund zwischen dem Knochen und dem Implantat entstehen. Dieser Verbund soll nicht nur Druck-, sondern auch Scher- und Zugkräfte übertragen können.

In der älteren DE-OS 35 19 073 ist ein künstliches knochenerzeugendes Biomaterial vorgeschlagen, das ein Gemisch aus einem knochenerzeugenden Faktor und einem ersten Träger für den Faktor enthält, wobei der Träger ausgewählt wird unter Collagen, seinen Derivaten und denaturierten Substanzen. Dieses Biomaterial kann auf einen als Formkörper ausgebildeten zweiten Träger aus Keramikmaterial, Metall oder synthetischem Harz aufgestrichen werden.

Aufgabe der Erfindung ist es, ein Implantat der eingangs genannten Art zu schaffen, durch welches Biokompatibilität und mechanische Fertigkeit und ein echter Verbund zwischen dem körpereigenen Gewebe am Implantationsort und dem Implantat gewährleistet wird.

Diese Aufgabe wird beim eingangs genannten Implantat erfindungsgemäß dadurch gelöst, daß der bioaktive Werkstoff Wachstumsfaktoren für menschliche oder tierische Zellen aufweist, die kovalent an der bindungsaktive Gruppen aufweisenden Oberfläche einer am Implantat-Grundkörper vorhandenen Polymerschicht gebunden sind.

Unter dem Begriff "Implantat" werden nicht nur Prothesen verstanden, sondern auch künstliche Organe, beispielsweise künstliche Nieren, Gefäße, Hautersatz, künstliche Augenlinsen, sogenannte Intraokularlinsen, Zahnersatz und auch Kontaktlinsen.

Durch die angegebene Beschichtung mit Wachstumsfaktoren und gegebenenfalls menschlichen bzw. tierischen Zellen wird eine verbesserte Verträglichkeit des Implantats erzielt. Es lassen sich auch Hornhautimplantate mit Epithel- bzw. Endothelzellbeschichtung herstellen. Man erreicht eine hohe Biokompatibilität, und das Einwachsen des Implantats ist gewährleistet. Durch die Erfindung wird auch der therapeutische Einsatz von Kunststoffen, z.B. in Form extrakorporaler oder implantierbarer künstlicher Organe und Gefäße ermöglicht. Durch die auf der Grundkörperoberfläche vorhandene Polymerschicht wird außerdem ein Schutz, insbesondere Korrosionsschutz, für einen metallischen Implantatgrundkörper erzielt.

Bei den Wachstumsfaktoren kann es sich um Stoffwechselprodukte menschlicher oder tierischer Zellen handeln. Auch Blutserum oder Bestandteile davon, insbesondere Fibronectin, eignen sich als Wachstumsfaktoren. Ferner kann eine Kombination aus Stoffwechselprodukten von Zellen und auf biochemischem Weg modifizierten Naturprodukten als Wachstumsfaktor zum Einsatz kommen. Ferner eignen sich als Wachstumsfaktoren Proteine, die zur Zelldifferenzierung führen. Es kann sich hierbei um Proteine mit aktiver Knocheninduktion (J. Dent. Res. (1971), Seiten 1392-1406) handeln, die in Form einer Knochenpaste (Knochensubstanz induzierende Proteine und z. B. Kollagen),vorliegen. Die Wachstumsfaktoren können dabei als Knochenpuderextrakt ausgebildet sein. Das Knochenpuderextrakt kann dabei mit einer extrazellulären Matrix (kollagenhaltig) an die Polymerisatoberfläche gebunden sein. Hierzu können polyklonale Antikörper an die Polymeroberfläche gebunden sein, die gegen die Knochensubstanz induzierenden Proteine und/oder die extrazelluläre Matrix gerichtet sind. Auch eignet sich ein Kollagen-Apatit-Mischpräparat (Z. Orthop.121 (1983), S. 115-123), bei dem das Kollagen kovalent an die Polymeroberfläche gebunden ist.

Als bindungsaktive Gruppen kann die Polymeroberfläche Oxirangruppen aufweisen. Andere Mechanismen zur kovalenten Bindung sind aus "Chemikerzeitung", 97. Jahrgang (1973, Nr. 11, Seite 612) bekannt. Bei dem Polymer kann es sich um ein Copolymerisat handeln, das aus wenigstens zwei der Monomere Methacrylamid, N-Methylen-bis-Methacrylamid, Allylglycidylether oder Glycidylmethacrylat gebildet sein kann. Der Grundkörper, auf welchem die polymere Schicht aufgebracht ist, kann je nach Verwendung aus Metall oder Kunststoff bestehen. Zur besseren Verankerung können Vorsprünge am Grundkörper vorgesehen sein.

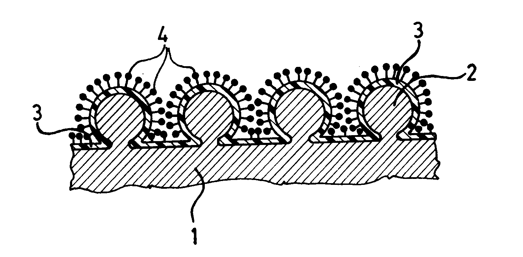

Anhand der beiliegenden Figur wird die Erfindung noch näher erläutert.

Die Figur zeigt einen Teilausschnitt aus einer Oberfläche eines Implantats. Auf der Implantatoberfläche sind Wachstumsfaktoren für menschliche und/oder tierische Zellen vorhanden. Es kann sich hier beispielsweise um ein Hüftgelenkimplantat handeln. Ein Grundkörper 1 dieses Implantats besteht aus Metall. Verankerungsvorsprünge 2, beispielsweise in Form von aufgesinterten Kugeln, dienen zur besseren Verankerung und zur Vergrößerung der Implantatoberfläche. Die Vorsprünge können auch anders geformt sein, wobei es von Vorteil ist, daß durch ihre Form Hinterschneidungen gebildet werden, die eine einwandfreie Verankerung für die Polymerschicht und auch für die Zellen, über welche eine Verbindung mit dem entsprechenden Gewebe hergestellt werden soll, gewährleisten.

Auf die Oberflächen des Grundkörpers 1 und der Verankerungsvorsprünge 2 ist eine Polymerschicht 3 aufgebracht, deren Oberfläche bindungsaktive Gruppen für eine kovalente Bindung von Wachstumsfaktoren 4 aufweist. Bei den bindungsaktiven Gruppen handelt es sich bevorzugt um Oxirangruppen. Die Wachstumsfaktoren, welche durch kovalente Bindung an der Polymeroberfläche gebunden sind, werden ausgewählt in Abhängigkeit vom Verwendungszweck des Implantats. Wenn es sich um ein Knochen ersetzendes Implantat, beispielsweise für ein Hüftgelenk, handelt, werden Knochensubstanz induzierende Proteine (J. Dent. Res. 50 (1971), Seiten 1392 bis 1406) als Wachstumsfaktoren gewählt. Bereits Spuren dieses Proteins genügen, um mesenchymale Zellen zur Differenzierung zu knochenbildenden Zellen anzuregen.

Im folgenden werden einige Beispiele für die Immobilisierung von Proteinen beschrieben.

### Beispiel 1

Aminogruppen tragende Trägeroberflächen lassen sich z. B. mit Thiophosgen aktivieren. Als aktivierende Spezie entsteht dabei ein Isothiocyanat am Träger.

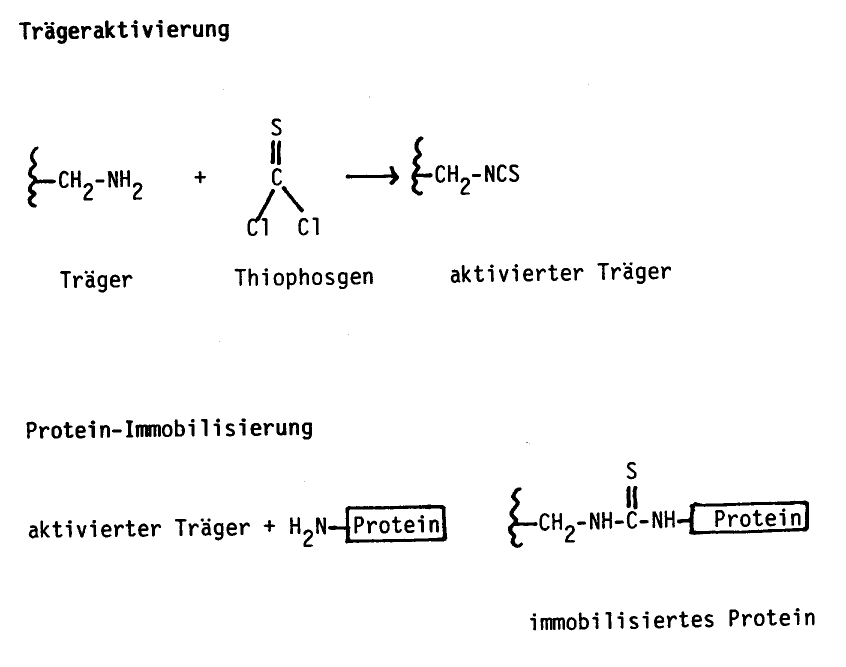

### Beispiel 2

Ein breites Reaktionsspektrum bietet die Epichlorhydrinmethode. Bei ihr wird die Trägeroberfläche mit Hilfe von Epichlorhydrin mit oxirangruppenhaltigem Spacern gespickt. Die unter Ringspannung stehenden Epoxygruppen lassen sich unter sehr milden Reaktionsbedingungen mukleophil mit Thiol-, Hydroxyl- oder Aminogruppen öffnen.

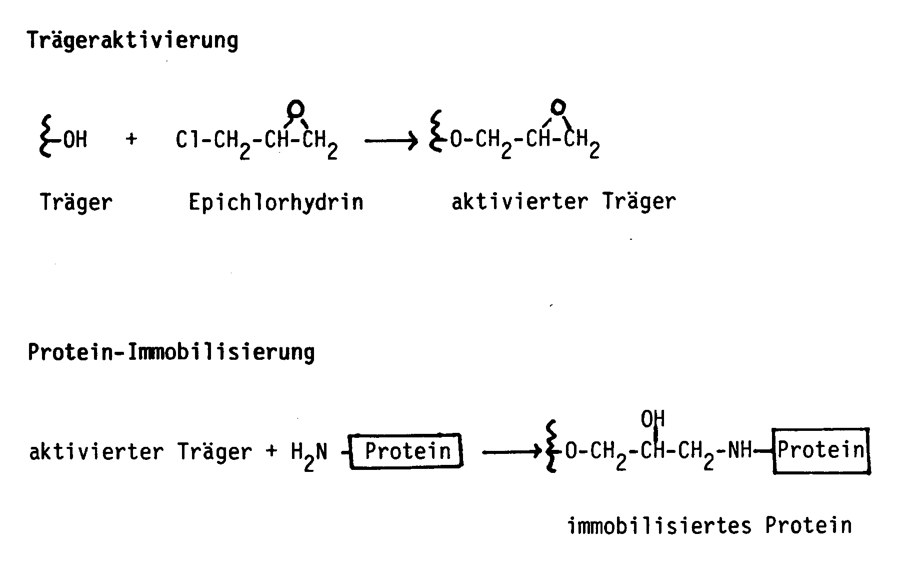

### Beispiel 3

Die Effektivität einer Immobilisierung hängt nicht nur von der Wahl der Verknüpfungsmethode, sondern auch vom Abstand Träger zu Protein ab.

Solchen Gesichtspunkten trägt z. B. die Bisepoxioxiran-Methode Rechnung. Hierbei wird eine Oxirangruppe zur Verankerung am Träger und die zweite zur Fixierung des Proteins verwandt.

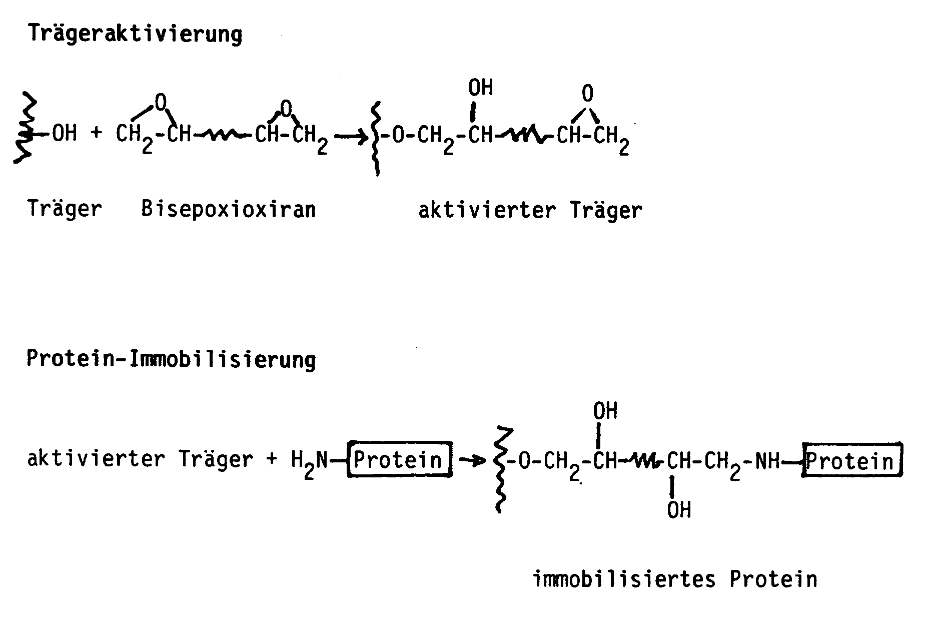

## Patentansprüche

1. Implantat mit einem Grundkörper, der einen Oberflächenüberzug aus einem bioaktiven Werkstoff aufweist, dadurch gekennzeichnet, daß der bioaktive Werkstoff Wachstumsfaktoren für menschliche oder tierische Zellen aufweist, die kovalent an bindungsaktive Gruppen (Isothiocyanat, Oxiran) der Oberfläche eines auf den Grundkörper aufgebrachten Polymers gebunden sind.

2. Implantat nach Anspruch 1, dadurch gekennzeichnet,
daß die Wachstumsfaktoren Stoffwechselprodukte menschlicher und tierischer Zellen sind.

3. Implantat nach Anspruch 1, dadurch gekennzeichnet,
daß die Wachstumsfaktoren Blutserum oder Bestandteile davon sind.

4. Implantat nach Anspruch 3, dadurch gekennzeichnet, daß die Wachstumsfaktoren Fibronectin sind.

5. Implantat nach Anspruch 1, dadurch gekennzeichnet,
daß die Wachstumsfaktoren eine Kombination aus Stoffwechselprodukten von Zellen und auf biochemischem Weg modifizierten Naturprodukten sind.

6. Implantat nach Anspruch 1, dadurch gekennzeichnet,
daß die Wachstumsfaktoren zur Zelldifferenzierung führende Proteine sind.

7. Implantat nach Anspruch 1 oder 6, dadurch gekennzeichnet,
daß die Wachstumsfaktoren Knochensubstanz induzierende Proteine sind.

8. Implantat nach Anspruch 6 oder 7, dadurch gekennzeichnet,
daß die Wachstumsfaktoren als Knochenpuderextrakt ausgebildet sind.

9. Implantat nach einem der Ansprüche 6 bis 8, dadurch gekennzeichnet,
daß das Knochenpuderextrakt mit einer extrazellulären Matrix an die Polymeroberfläche gebunden ist.

10. Implantat nach einem der Ansprüche 6 bis 9, dadurch gekennzeichnet,
daß an der Polymeroberfläche polyklonale Antikörper gebunden sind, die gegen die Knochensubstanz induzierende Proteine gerichtet sind.

11. Implantat nach Anspruch 1, dadurch gekennzeichnet,
daß die Polymeroberfläche Oxirangruppen aufweist.

12. Implantat nach Anspruch 1, dadurch gekennzeichnet,
daß das Polymer ein Copolymerisat ist aus wenigstens zwei der Monomere Methacrylat, N-Methylen-bis-Methacrylamid, Allylglycidylether und Glycidylmethacrylat.

13. Implantat nach einem der Ansprüche 1 bis 12, dadurch gekennzeichnet,
daß der Grundkörper aus Metall besteht.

14. Implantat nach Anspruch 13, dadurch gekennzeichnet,
daß der Grundkörper an seiner Oberfläche Hinterschneidungen bildende Verankerungsvorsprünge aufweist.

## Claims

1. An implant with a base body which has a surface coating comprising a bioactive material, characterised in that the bioactive material has growth factors for human or animal cells which are covalently bonded to bonding-active groups (isothiocyanate, oxirane) of the surface of a polymer applied to the base body.

2. An implant according to claim 1 characterised in that the growth factors are metabolism products of human and animal cells.

3. An implant according to claim 1 characterised in that the growth factors are blood serum or constituents thereof.

4. An implant according to claim 3 characterised in that the growth factors are fibronectin.

5. An implant according to claim 1 characterised in that the growth factors are a combination of cell metabolism products and biochemically modified natural products.

6. An implant according to claim 1 characterised in that the growth factors are proteins leading to cell differentiation.

7. An implant according to claim 1 or claim 6 characterised in that the growth factors are bone substance-inducing proteins.

8. An implant according to claim 6 or claim 7 characterised in that the growth factors are in the form of bone powder extract.

9. An implant according to one of claims 6 to 8 characterised in that the bone powder extract is bonded with an extra-cellular matrix to the polymer surface.

10. An implant according to one of claims 6 to 9 characterised in that bonded to the polymer surface are polyclonal antibodies which are directed against the bone substance-inducing proteins.

11. An implant according to claim 1 characterised in that the polymer surface has oxirane groups.

12. An implant according to claim 1 characterised in that the polymer is a copolymer comprising at least two of the monomers methacrylate, N-methylene-bis-methacrylamide, allylglycidylether and glycidylmethacrylate.

13. An implant according to one of claims 1 to 12 characterised in that the base body comprises metal.

14. An implant according to claim 13 characterised in that the base body has anchoring projections forming undercut configurations at the surface of the base body.

## Revendications

1. Implant avec un corps de base qui présente un revêtement superficiel composé d'une matière bioactive, caractérisé en ce que la matière bioactive comporte des facteurs de croissance pour cellules humaines ou animales, lesquels facteurs de croissance sont liés par covalence à des groupes (isothiocyanate, oxiranne), actifs dans la formation de liaisons, de la surface d'un polymère appliqué sur le corps de base.

2. Implant selon la revendication 1, caractérisé en ce que les facteurs de croissance sont des produits du métabolisme de cellules humaines ou animales.

3. Implant selon la revendication 1, caractérisé en ce que les facteurs de croissance sont du sérum sanguin ou des éléments constitutifs de celui-ci.

4. Implant selon la revendication 3, caractérisé en ce que les facteurs de croissance sont de la fibronectine.

5. Implant selon la revendication 1, caractérisé en ce que les facteurs de croissance sont une combinaison de produits du métabolisme de cellules et de produits naturels modifiés par voie biochimique.

6. Implant selon la revendication 1, caractérisé en ce que les facteurs de croissance sont des protéines conduisant à la différenciation de cellules.

7. Implant selon la revendication 1 ou 6, caractérisé en ce que les facteurs de croissance sont des protéines favorisant la formation de substance osseuse.

8. Implant selon la revendication 6 ou 7, caractérisé en ce que les facteurs de croissance sont présentés sous la forme d'extrait de poudre d'os.

9. Implant selon l'une quelconque des revendications 6 à 8, caractérisé en ce que l'extrait de poudre d'os est lié, au moyen d'une matrice extra-cellulaire, à la surface du polymère.

10. Implant selon l'une quelconque des revendications 6 à 9, caractérisé en ce qu'à la surface du polymère sont liés des anticorps polyclonaux qui sont dirigés contre les protéines favorisant la formation de substance osseuse.

11. Implant selon la revendication 1, caractérisé en ce que la surface du polymère comporte des groupes oxiranne.

12. Implant selon la revendication 1, caractérisé en ce que le polymère est un copolymérisat composé d'au moins deux des monomères suivants : méthacrylamide, N-méthylène-bis-méthacrylamide, éther allylglycidylique et méthacrylate de glycidyle.

13. Implant selon l'une quelconque des revendications précédentes, caractérisé en ce que le corps de base est en métal.

14. Implant selon la revendication 13, caractérisé en ce que le corps de base présente à sa surface des saillies d'ancrage formant des contre-dépouilles.
